(19)

Europäisches
Patentamt
European
Patent Office
Office européen
des brevets

(11) **EP 4 310 175 A1**

(12) **EUROPEAN PATENT APPLICATION**
published in accordance with Art. 153(4) EPC

(43) Date of publication:
**24.01.2024 Bulletin 2024/04**

(21) Application number: **22771455.7**

(22) Date of filing: **16.03.2022**

(51) International Patent Classification (IPC):
*C12N 5/071* (2010.01)   *C12N 5/10* (2006.01)
*C12N 1/00* (2006.01)   *C12P 21/00* (2006.01)

(52) Cooperative Patent Classification (CPC):
**C12N 1/00; C12N 5/06; C12N 5/10; C12P 21/00**

(86) International application number:
**PCT/JP2022/011766**

(87) International publication number:
**WO 2022/196710 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2021 JP 2021043346**

(71) Applicant: **FUJIFILM Corporation**
**Tokyo 106-8620 (JP)**

(72) Inventors:
• **INADA, Atsushi**
 **Ashigarakami-gun, Kanagawa 258-8577 (JP)**
• **TANIGUCHI, Kosuke**
 **Ashigarakami-gun, Kanagawa 258-8577 (JP)**
• **TAKAHASHI, Naoto**
 **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **CELL CULTURING METHOD, AND METHOD FOR PRODUCING USEFUL SUBSTANCE**

(57)    An object to be achieved by the present invention is to provide a culture method for cells, which makes it possible to suppress oxygen deficiency of cells in a case where the cell is cultured at a high density, and a production method for a useful substance using the culture method for cells. According to the present invention, there is provided a culture method for cells, in which a relationship between a stirring power P/V per unit volume, a dissolved oxygen concentration CL0, and a viable cell density VCD satisfies a condition of Expression 1, in cell culture in which the viable cell density is $60 \times 10^6$ cells/mL or more.

$$\text{Expression 1: } 0.0059 \times \exp[0.0243 \times (P/V)] \times CL0/VCD \geq 1.10 \times 10^{-17}$$

In the expression, L0 indicates a dissolved oxygen concentration [mol/mL]; VCD indicates a viable cell density [cells/mL]; and P/V indicates a stirring power [W/m$^3$] per unit volume, which is defined by a predetermined expression.

EP 4 310 175 A1

**Description**

Technical Field

The present invention relates to a culture method for cells, in which oxygen deficiency of cells is suppressed. The present invention further relates to a production method for a useful substance, using the above-described culture method for cells.

Background Art

Oxygen supply to cells is very important in cell culture, and in a case where the amount of oxygen supply is insufficient, the cells are in a state of oxygen deficiency, which causes stagnation of proliferation and a decrease in the productivity of useful substances. In general, for the oxygen supply capacity of a cell culture device, kLa (gas-liquid oxygen transfer capacity coefficient), which is a parameter indicating the rate (magnitude) of the dissolution of oxygen from air bubbles into a culture solution is used as an indicator. In the related art, studies of obtaining a sufficient dissolved oxygen concentration by increasing kLa (increasing the dissolution rate of air bubble oxygen into a culture medium) in response to an increase in a required oxygen amount in association with an increase in cell density have been made.

Patent Document 1 describes a culture method for a biological cell characterized by measuring a dissolved oxygen concentration in a culture solution in a culture tank at a plurality of positions in the space inside the culture tank and determining a stirring speed for the culture solution in the culture tank based on a difference between a plurality of measured dissolved oxygen concentrations. The culture method described in JP2011-92117A is a method of controlling a stirring speed of a culture solution with high accuracy, particularly in a case of culturing a biological cell using a microcarrier to sustain the culture of the biological cell for a long period of time.

Prior Art Documents

Patent Document

Patent Document 1: JP2011-92117A

**SUMMARY OF THE INVENTION**

However, it was found that there arises a problem that in a case where cell density is high, cells fall into an oxygen deficiency state even though the dissolved oxygen concentration in the culture medium is sufficient. An object to be achieved by the present invention is to provide a culture method for cells, which makes it possible to suppress oxygen deficiency of cells in a case where the cell is cultured at a high density. Further, an object to be achieved by the present invention is to provide a production method for a useful substance using the culture method for cells.

As a result of studying the cause of the above-described problem that in a case where the cell density is high, cells fall into an oxygen deficiency state even though the dissolved oxygen concentration in the culture medium is sufficient, the inventors of the present invention found that in a case where the cell density is high, the oxygen supply rate from the culture medium to the cells decreases, which results in oxygen deficiency. As a result of diligent studies to solve the above problem, the inventors of the present invention found that in a case where a relationship between a stirring power P/V per unit volume, a dissolved oxygen concentration CL0, and a viable cell density VCD satisfies a predetermined condition, the oxygen deficiency in high-density culture is ameliorated, which enables stable culture. Further, the inventors of the present invention found that even in a case where a relationship between a newly defined oxygen transfer capacity coefficient (kcella) in terms of the transfer from a culture medium to cells, a dissolved oxygen concentration CL0, and a viable cell density VCD satisfies a predetermined condition, the oxygen deficiency in high-density culture is ameliorated, which enables stable culture. The present invention has been completed based on the above findings.

That is, according to the present invention, the following inventions are provided.

<1> A culture method for cells, in which a relationship between a stirring power P/V per unit volume, a dissolved oxygen concentration CL0, and a viable cell density VCD satisfies a condition of Expression 1, in cell culture in which the viable cell density is $60 \times 10^6$ cells/mL or more,

$$\text{Expression 1: } 0.0059 \times \exp[0.0243 \times (P/V)] \times CL0/VCD \geq 1.10 \times 10^{-17}$$

in the expression,

CLO indicates a dissolved oxygen concentration [mol/mL];
VCD indicates a viable cell density [cells/mL]; and
P/V indicates a stirring power [W/m$^3$] per unit volume, which is defined by the following expression;

$$P/V = \rho \times (n/60)^3 \times Di^5/V \times Np$$

in the expression,
$\rho$ indicates a density of a culture solution [kg/m$^3$];
n indicates a stirring rotation speed [rpm];
Di indicates a stirring blade diameter [m];
V indicates a culture solution amount [m$^3$]; and
Np indicates a stirring power coefficient.

<2> The culture method for cells according to <1>, in which the stirring power P/V per unit volume satisfies, 60 ≤ P/V ≤ 2,000.

<3> The culture method for cells according to <1> or <2>, in which the stirring rotation speed n satisfies, 120 ≤ n ≤ 600.

<4> The culture method for cells according to any one of <1> to <3>, in which the relationship between the stirring power P/V per unit volume, the dissolved oxygen concentration CL0, and the viable cell density VCD satisfies a condition of $0.0059 \times \exp[0.0243 \times (P/V)] \times CL0/VCD \leq 4.00 \times 10^{-17}$.

<5> The culture method for cells according to any one of <1> to <4>, in which a cell culture period satisfying the condition of Expression 1 is 20 days or more.

<6> The culture method for cells according to any one of <1> to <5>, in which the condition of Expression 1 is satisfied over an entire culture period of cells.

<7> A culture method for cells, in which a relationship between kcella defined as below, a dissolved oxygen concentration CL0 in a steady state, and a viable cell density VCD satisfies a condition of Expression 2, in cell culture in which the viable cell density is $60 \times 10^6$ cells/mL or more,

$$\text{Expression 2: } kcella \times CL0/VCD \geq 1.00 \times 10^{-17} \text{ [mol/s/cell]}$$

in the expression,

CLO indicates a dissolved oxygen concentration [mol/mL];
VCD indicates a viable cell density [cells/mL]; and
the kcella indicates an overall oxygen transfer capacity coefficient [/s] from a culture medium to cells, which is defined according to $Ln(CL/CLO) = -kcella \times t$,
in the expression,
-kcella is defined as a slope which is obtained by defining a steady state as a state in which a culture solution is continuously subjected to stirring and oxygen supply to maintain a dissolved oxygen concentration of a predetermined set value CL0 in the culture solution for 24 hours or more, defining a temporal starting point as a moment at which the oxygen supply is stopped while the stirring is continued from the steady state, measuring a change between a dissolved oxygen concentration CLO at the starting point and a dissolved oxygen concentration CL in the solution for 60 seconds from the starting point, and subjecting a change in Ln(CL/CLO) with respect to an elapsed time t to linear approximation according to a least square method.

<8> The culture method for cells according to <7>, in which the kcella satisfies, 0.03 ≤ kcella ≤ 0.39.

<9> The culture method for cells according to <7> or <8>, in which the relationship between the kcella, the dissolved oxygen concentration CL0 in the steady state, and the viable cell density VCD satisfies a condition of kcella × CLO/VCD ≤ $4.00 \times 10^{-17}$ [mol/s/cell].

<10> The culture method for cells according to any one of <7> to <9>, in which culture is controlled such that the condition of Expression 2 is satisfied by grasping, in advance in a target culture tank and at a target liquid amount, a correlation between a calculated value of a turbulence energy dissipation rate $\varepsilon$ of the culture solution and an actually measured value of the kcella in a case where a stirring rotation speed is changed and increasing a stirring power in association with cell proliferation to increase kcella obtained from the correlation.

<11> The culture method for cells according to any one of <7> to <9>, in which culture is controlled such that the condition of Expression 2 is satisfied by increasing at least one of the kcella or the CLO in association with an increase of the VCD.

<12> The culture method for cells according to any one of <7> to <11>, in which a cell culture period satisfying the condition of Expression 2 is 20 days or more.

<13> The culture method for cells according to any one of <7> to <12>, in which the condition of Expression 2 is satisfied over an entire culture period of cells.

<14> The culture method for cells according to any one of <1> to <13>, in which the CLO satisfies, $0.45 \times 10^{-8} \leq$ CLO $\leq 6.75 \times 10^{-8}$.

<15> The culture method for cells according to any one of <1> to <14>, in which the viable cell density is $80 \times 10^6$ cells/mL or more.

<16> The culture method for cells according to any one of <1> to <15>, in which the culture solution amount is 50 L or more.

<17> The culture method for cells according to any one of <1> to <16>, in which the culture method for cells is perfusion culture.

<18> The culture method for cells according to any one of <1> to <17>, in which the cells are mammalian cells.

<19> The culture method for cells according to <18>, in which the mammalian cell is a Chinese hamster ovary cell.

<20> The culture method for cells according to any one of <1> to <19>, in which an average cell size of the cells is 12 $\mu$m or more.

<21> The culture method for cells according to any one of <1> to <20>, in which during the cell culture, a concentration of lactic acid in a culture solution is less than 1.2 g/L.

<22> The culture method for cells according to any one of <1> to <21>, in which during the cell culture, a concentration of lactate dehydrogenase in a culture solution is less than 2,000 U/L.

<23> A production method for a useful substance, comprising culturing cells by the culture method for cells according to any one of <1> to <22> to cause the cells to produce a useful substance.

<24> The production method for a useful substance according to <23>, in which the useful substance is a recombinant protein.

According to the culture method for cells according to the aspect of the present invention, oxygen deficiency of cells can be suppressed even in a case where cells are cultured at a high density. The culture method for cells according to the aspect of the present invention is useful in the production of a useful substance.

## BRIEF DESCRIPTION OF THE DRAWINGS

Fig. 1 is a view illustrating a relationship between a rate of molecular diffusion of oxygen transfer, an overall oxygen transfer capacity coefficient (defined as kcella), and a concentration difference between an oxygen concentration in a culture tank and an oxygen concentration on a cell surface (CLO - Ccell).
Fig. 2 is a schematic view in a case where the viable cell density is low.
Fig. 3 is a schematic view in a case where the viable cell density is high.
Fig. 4 is a schematic view in a case where kcella is increased in a case where the viable cell density is high.
Fig. 5 is a view illustrating a measuring method for the kcella.
Fig. 6 is a view illustrating a cell proliferation period and an antibody production period in Examples.
Fig. 7 is a graph showing a relationship between the kcella and $0.0059 \times \exp[0.0243 \times (P/V)] \times CLO/VCD \times 10^{17}$.
Fig. 8 is a graph showing a relationship between the kcella and kcella $\times CL0/VCD \times 10^{17}$.
Fig. 9 is a graph showing a relationship between a turbulence energy dissipation rate $\varepsilon$ and the kcella.

## DESCRIPTION OF THE PREFERRED EMBODIMENTS

Hereinafter, the contents of the present invention will be described in detail. The numerical value range expressed using the symbol "~" according to the present specification means a range including the numerical values described before and after the symbol "~" as the minimum value and the maximum value, respectively. In the present specification, s for indicating a unit indicates a second.

The present invention relates to a culture method for cells, in which a relationship between a stirring power P/V per unit volume, a dissolved oxygen concentration CL0, and a viable cell density VCD satisfies a predetermined condition (a condition of Expression 1 described later), or a relationship between a kcella defined as below, a dissolved oxygen concentration CLO in a steady state, and a viable cell density VCD satisfies a predetermined condition (a condition of Expression 2 described later), in cell culture in which the viable cell density is $60 \times 10^6$ cells/mL or more.

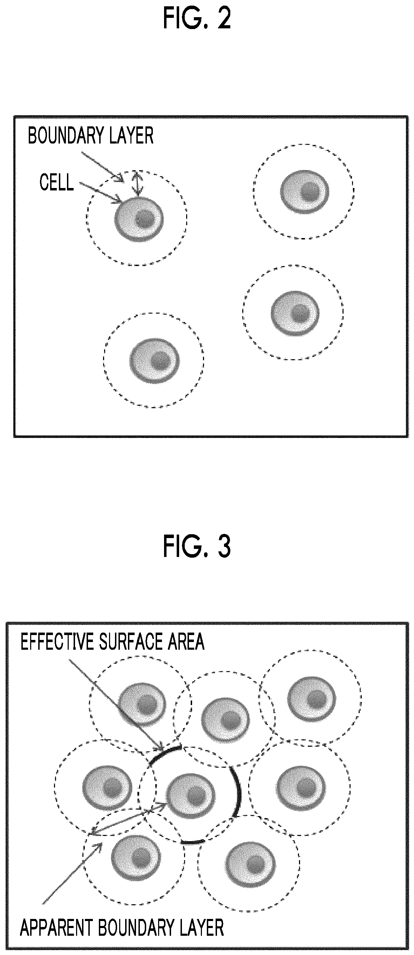

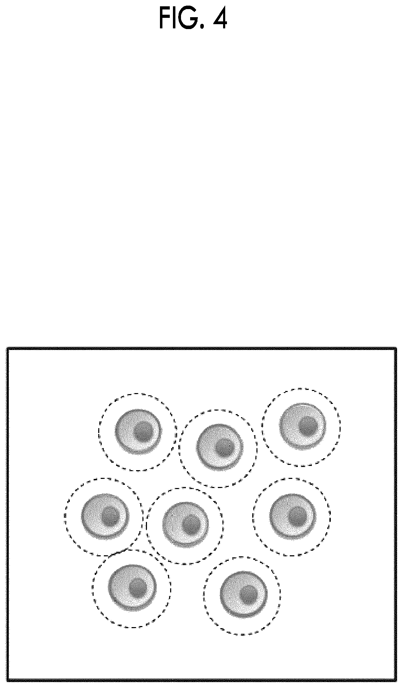

JP2011-92117A describes that, as Example, oxygen concentration distributions (see Fig. 3 and Fig. 4) in the proliferation in which a range of the viable cell density is $7 \times 10^6$ cells/mL or less are controlled by the stirring rotation speed. However, the range of the viable cell density specified in JP2011-92117A is different from the viable cell density defined in the present invention. It is noted that in the adhesion culture including a microcarrier described in JP2011-92117A, the viable cell density is generally $10 \times 10^6$ cells/mL or less, which is different from the viable cell density specified in the present invention.

<Oxygen supply rate between culture medium and cells and mechanism of oxygen deficiency>

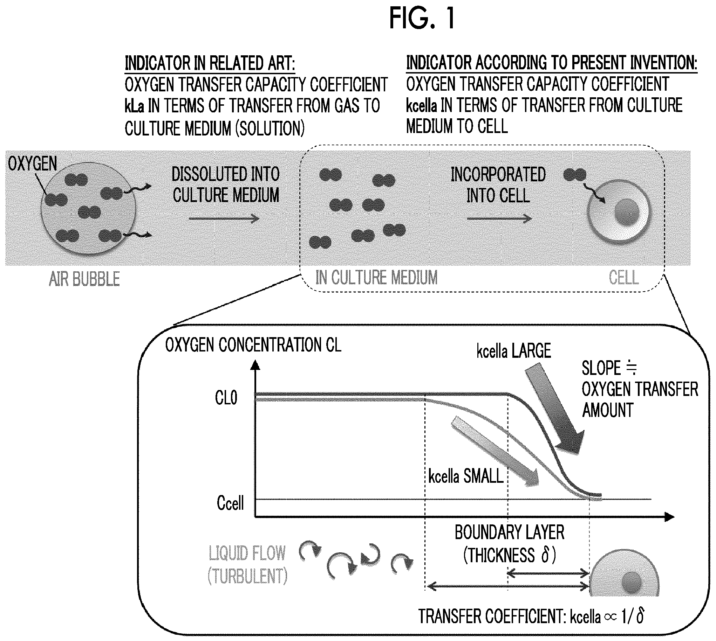

Even in a dynamic culture method, it is conceived that a layer (a boundary layer) in which a culture solution stays is present around a cell.
Here, in the oxygen supply system for cell culture, the oxygen supply amount from the culture medium to the cells is proportional to each of the following (1) and (2) (Fig. 1) in the boundary layer in which the culture solution stays around the cell.

    (1) The overall oxygen transfer capacity coefficient (defined as kcella). The thickness of the boundary layer depends on the fluidity state of the surrounding culture medium.
    (2) The concentration difference between the oxygen concentration in the surrounding culture tank and the oxygen concentration on the cell surface (CLO - Ccell)

In a case where the viable cell density is low, the distance between cells is sufficiently large, and kLa, which is a transfer capacity coefficient for dissolution of oxygen from air bubbles into a culture medium, is sufficiently high. That is, in a case where the dissolved oxygen concentration CLO is sufficiently high, the oxygen supply from the culture medium to the cells, which is expressed by kcella $\times$ (CLO - Ccell), is sufficiently carried out, and thus oxygen deficiency does not occur (Fig. 2).
It was found that in a case where the viable cell density is high, oxygen deficiency occurs even in a case where the dissolved oxygen concentration is sufficiently increased depending on the viable cell density. In a case where the viable cell density is high, the relative distance between the cells in the culture tank is reduced. Even in a case where the distance between the cells is reduced, the adjacent cells do not directly overlap each other; however, the boundary layers of the adjacent cells overlap each other. In a case where a portion where the boundary layers overlap is generated between adjacent cells, a boundary layer of another cell exists around each of the cell, and thus the apparent boundary layer thickness increases, and the kcella that is inversely proportional to the thickness of the boundary layer is reduced. In addition, the surface area effective for oxygen transfer is reduced, and thus the kcella is reduced. As a result, even in a case where the dissolved oxygen concentration ($\approx$ kLa) is sufficiently high, it is conceived that the oxygen transfer amount (rate) expressed by kcella $\times$ (CLO - Ccell) decreases and thus oxygen deficiency occurs (Fig. 3).
In such a case where the viable cell density is high, in a case of increasing the kcella, the overlap of the boundary layers is eliminated even in a case where the distance between the cells is small, and it is possible to achieve a sufficient oxygen supply (Fig. 4).

<First aspect according to present invention>

In the culture method for cells according to the first aspect of the present invention, a relationship between a stirring power P/V per unit volume, a dissolved oxygen concentration CL0, and a viable cell density VCD satisfies a condition of Expression 1 below.

$$\text{Expression 1: } 0.0059 \times \exp[0.0243 \times (P/V)] \times CL0/VCD \geq 1.10 \times 10^{-17}$$

in the expression,

    CLO indicates a dissolved oxygen concentration [mol/mL];
    VCD indicates a viable cell density [cells/mL]; and
    P/V indicates a stirring power [W/m$^3$] per unit volume, which is defined by the following expression;

$$P/V = \rho \times (n/60)^3 \times Di^5/V \times Np$$

in the expression,

$\rho$ indicates a density of a culture solution [kg/m$^3$];
n indicates a stirring rotation speed [rpm];
Di indicates a stirring blade diameter [m];
V indicates a culture solution amount [m$^3$]; and
Np indicates a stirring power coefficient.

The stirring power coefficient can be calculated based on the physical properties of the liquid (the viscosity and the density), the information on the culture tank/stirring blade (the number of stirring blades, the stirring blade width, the stirring blade diameter, and the inner diameter of the culture tank), and the information on the stirring rotation speed. Specifically, the stirring power coefficient can be determined according to the following expression.

$$\text{Stirring Power coefficient Np} = A/Re + B \times \{(10^3 + 1.2 \times Re^{0.66})/(10^3 + 3.2 \times Re^{0.66})\}^p \times (H/D)^{(0.35 + a)}$$

$a = np \times h/2/D$
$b = d/D$
$A = 14 + a \times \{670 \times (b - 0.6)^2 + 185\}$
$B = 10^{\{1.3 - 4 \times (a-0.5)(a-0.5) - 1.14 \times b\}}$
$p = 1.1 + 4 \times a - 2.5 \times (b - 0.5)^2 - 7 \times a^4$
$Re = n/60 \times d^2 \times \rho/\mu$
np: number of stirring blades [blades]
h: stirring blade width [m]
d: stirring blade diameter [m]
n: stirring rotation speed [rpm]
D: inner diameter of culture tank [m]
H: depth of liquid [m]
p: liquid density [kg/m$^3$]
$\mu$: liquid viscosity [Pa-s]

The stirring power P/V per unit volume preferably satisfies, $60 < P/V < 2{,}000$, more preferably satisfies $60 \leq P/V \leq 1{,}500$, still more preferably satisfies $60 \leq P/V \leq 1{,}000$, particularly preferably satisfies $60 \leq P/V \leq 500$, and most preferably satisfies $60 \leq P/V \leq 200$.

The stirring rotation speed n preferably satisfies, $100 \leq n \leq 600$, more preferably satisfies, $110 \leq n \leq 400$, and still more preferably satisfies, $120 \leq n \leq 300$.

The relationship between the stirring power P/V per unit volume, the dissolved oxygen concentration CL0, and the viable cell density VCD preferably satisfies a condition of $0.0059 \times \exp[0.0243 \times (P/V)] \times CL0/VCD \leq 4.00 \times 10^{-17}$.

The lower limit of $0.0059 \times \exp[0.0243 \times (P/V)] \times CL0/VCD$ may be $1.20 \times 10^{-17}$ or more, $1.30 \times 10^{-17}$ or more, $1.40 \times 10^{-17}$ or more, or $1.50 \times 10^{-17}$ or more.

The upper limit of $0.0059 \times \exp[0.0243 \times (P/V)] \times CL0/VCD$ may be $3.50 \times 10^{-17}$ or less, or $3.00 \times 10^{-17}$ or less.

The cell culture period satisfying the condition of Expression 1 is preferably 10 days or more, more preferably 20 days or more, and still more preferably 30 days or more. It is particularly preferable that the condition of Expression 1 is satisfied over an entire culture period of cells.

<Second aspect according to present invention>

In the culture method for cells according to the second aspect of the present invention, a relationship between a kcella defined as below, a dissolved oxygen concentration CLO in a steady state, and a viable cell density VCD satisfies a condition of Expression 2 below.

$$\text{Expression 2: kcella} \times CL0/VCD \geq 1.00 \times 10^{-17} \text{ [mol/s/cell]}$$

in the expression,

CLO indicates a dissolved oxygen concentration [mol/mL];
VCD indicates a viable cell density [cells/mL]; and

the kcella indicates an overall oxygen transfer capacity coefficient [/s] from a culture medium to cells, which is defined according to Ln(CL/CLO) = -kcella × t,

where in the expression, -kcella is defined as a slope which is obtained by defining a steady state as a state in which a culture solution is continuously subjected to stirring and oxygen supply to maintain a dissolved oxygen concentration of a predetermined set value CLO in the culture solution for 24 hours or more, defining a temporal starting point as a moment at which the oxygen supply is stopped from the steady state, measuring a change between a dissolved oxygen concentration CLO at the starting point and a dissolved oxygen concentration CL in the solution for 60 seconds from the starting point, and subjecting a change in Ln(CL/CLO) with respect to an elapsed time t to linear approximation according to a least square method.

The kcella at the time when culture is carried out in a steady state can be calculated from a rate of decrease in the dissolved oxygen concentration in the culture solution where the rate of decrease is a rate in a case where only the oxygen supply is stopped while the stirring conditions and the culture temperature from the steady state being maintained.

Regarding the oxygen supply from the culture solution to the cells, it is conceived that the oxygen transfer in the boundary layer around the cell is rate-limiting or the cell respiration is rate-limiting; however, the boundary-membrane transfer is rate-limiting since the rate of decrease in the oxygen in the culture solution also changes depending on the stirring rotation speed during the measurement, in measuring the dissolved oxygen concentration in the culture solution by stopping the oxygen supply from the steady culture state. In addition, the fluidity state of the culture solution changes depending on the stirring rotation speed, and the thickness of the boundary layer around the cell also changes. In the boundary-membrane transfer rate-limiting step, the amount of oxygen required by the cell is larger than the oxygen transfer rate, and thus the oxygen consumption rate equals to the oxygen transfer rate in terms of the entire system (CL - Ccell ≈ CL is satisfied in a case where the oxygen concentration on the cell surface is denoted as Ccell [mol/mL]).

The expression for the oxygen transfer in the culture medium in this case is expressed as follows.

$$dCL/dt = -kcella \,(CL - Ccell) = -kcella \times CL$$

$$Ln(CL/CL0) = -kcella \times t$$

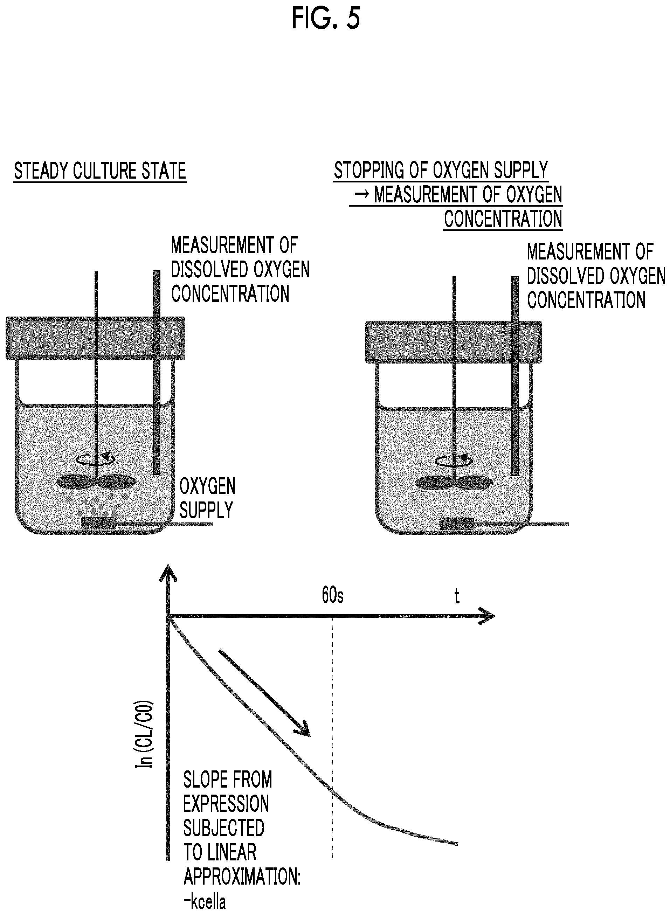

That is, the slope obtained by linearly approximating a curve at t = 0 to 60 [s] in a case of plotting the dissolved oxygen concentration Ln(CL/CL0) in the culture solution with respect to the elapsed time t is -kcella (Fig. 5).

The kcella preferably satisfies, 0.03 < kcella < 0.39. The lower limit value of the kcella may be 0.04 or more, 0.05 or more, or 0.10 or more. The upper limit value of the kcella may be 0.35 or less, 0.30 or less, or 0.26 or less.

The relationship between the kcella, the dissolved oxygen concentration CLO in the steady state, and the viable cell density VCD preferably satisfies a condition of kcella × CLO/VCD ≤ 4.00 × 10⁻¹⁷ [mol/s/cell].

The lower limit of kcella × CLO/VCD may be $1.10 \times 10^{-17}$ or more, $1.20 \times 10^{-17}$ or more, $1.30 \times 10^{-17}$ or more, $1.40 \times 10^{-17}$ or more, or $1.50 \times 10^{-17}$ or more.

The upper limit of kcella × CLO/VCD may be $3.50 \times 10^{-17}$ or less, $3.00 \times 10^{-17}$ or less, or $2.50 \times 10^{-17}$ or less.

In the present invention, culture may be controlled so that the condition of Expression 2 is satisfied by grasping, in advance in a target culture tank and at a target liquid amount, a correlation between a calculated value of a turbulence energy dissipation rate ε of the culture solution and an actually measured value of kcella in a case where a stirring rotation speed is changed and increasing a stirring power in association with cell proliferation (that is increasing the stirring rotation speed) to increase kcella obtained from the correlation.

The turbulence energy dissipation rate ε can be calculated by using a commercially available software in computational fluid dynamics (CFD) (for example, Fluent manufactured by ANSYS Inc.). The turbulence energy dissipation rate ε can be calculated, for example, by combining a mass conservation equation, a momentum conservation equation, a transport equation of the k-ε model, and an improved wall processing ε equation. The turbulence energy dissipation rate can be calculated by creating meshes that mimic the shape of the culture tank, the shape of the stirring blade, and the predetermined amount of liquid, setting, as parameters, the fluid viscosity, the fluid density, the stirring rotation speed, the rotation angle, the direction of gravitational force, and the liquid-surface boundary condition, and carrying out a convergence calculation.

In the present invention, culture may be controlled so that the condition of Expression 2 is satisfied by increasing at least one of the kcella or the CLO in association with an increase of the VCD.

The cell culture period satisfying the condition of Expression 2 is preferably 10 days or more, more preferably 20 days or more, and still more preferably 30 days or more. It is particularly preferable that the condition of Expression 2 is satisfied over an entire culture period of cells.

<In regard to culture method for cells according to embodiment of present invention>

As the culture medium that is used for cell culture, a culture medium that is generally used for culturing animal cells can be used. For example, CD OptiCHO (manufactured by Thermo Fisher Scientific, Inc.) medium, Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium is generally pH 6.0 to 8.0, preferably pH 6.8 to 7.6, and more preferably pH 7.0 to 7.4.

The culture temperature is generally 30°C to 40°C, preferably 32°C to 37°C, and more preferably 36°C to 37°C, and the culture temperature may be changed during the culture.

The culture is preferably carried out in an atmosphere having a $CO_2$ concentration of 0% to 40%, and preferably 2% to 10%.

The culture time is not particularly limited; however, it is generally 12 hours to 90 days, preferably 24 hours to 60 days, more preferably 24 hours to 30 days.

In the culture, the culture medium can be replaced, aerated, and stirred as necessary.

The culture method for cells according to the embodiment of the present invention can be carried out in a culture device (also referred to as a bioreactor) or other suitable containers. The culture can be carried out using, as the culture device, a fermenter tank type culture device, an air lift type culture device, a culture flask type culture device, a spinner flask type culture device, a microcarrier type culture device, a fluidized bed type culture device, a hollow fiber type culture device, a roller bottle type culture device, or the like.

In the culture method for cells according to the embodiment of the present invention, the culture solution amount is generally 1 L to 20,000 L and preferably 50 L or more, and it is, for example, 50 L to 2,000 L or 500 L to 2,000L.

In the culture method for cells according to the embodiment of the present invention, the dissolved oxygen concentration CLO (mol/mL) is preferably $0.45 \times 10^{-8} \leq CLO \leq 6.75 \times 10^{-8}$. The lower limit of the CLO may be $0.50 \times 10^{-8}$ mol/mL or more, $0.60 \times 10^{-8}$ mol/mL or more, $0.70 \times 10^{-8}$ mol/mL or more, $0.80 \times 10^{-8}$ mol/mL or more, or $0.90 \times 10^{-8}$ mol/mL or more. The upper limit of the CL0 may be $6.50 \times 10^{-8}$ mol/mL or less, $6.00 \times 10^{-8}$ mol/mL or less, $5.50 \times 10^{-8}$ mol/mL or less, or $5.00 \times 10^{-8}$ mol/mL or less.

In the culture method for cells according to the embodiment of the present invention, the viable cell density is $60 \times 10^6$ cells/mL or more, preferably $70 \times 10^6$ cells/mL or more, more preferably $80 \times 10^6$ cells/mL or more, and still more preferably $100 \times 10^6$ cells/mL or more. The viable cell density may be $120 \times 10^6$ cells/mL or more, $150 \times 10^6$ cells/mL or more, $200 \times 10^6$ cells/mL or more, or $300 \times 10^6$ cells/mL or more. The upper limit of the viable cell density is not particularly limited; however, it is generally $500 \times 10^6$ cells/mL or less.

The mode of the culture method for cells according to the embodiment of the present invention is not particularly limited, and the culture method may be, for example, any one of perfusion culture, batch culture, or fed-batch culture, where perfusion culture is preferable.

The batch culture is a discontinuous method in which cells are proliferated for a short period in a culture medium with a fixed volume and then completely harvested.

The fed-batch culture is a culture method that improves the batch process by a bolus supply or continuous supply of a culture medium, followed by replenishing the consumed components of the culture medium.

The perfusion culture is a culture method in which a fresh culture medium is added and concurrently, a used culture medium is removed, and thus it provides a possibility that the batch culture and the fed-batch culture can be further improved. In general, the perfusion culture makes it possible to achieve a high viable cell density. A typical perfusion culture begins with a batch culture start-up lasting 1 or 2 days, thereafter a fresh supplying culture medium is added to the culture product continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be continuous, stepwise, or intermittent, or a combination thereof. Animal cells are retained in the culture product and the used culture medium that is removed may substantially do not include cells or may have much fewer cells than the culture product. A useful substance expressed by cell culture can be retained in the culture product or harvested by the selection of the membrane pore diameter. To prevent the viable cell density during the culture from becoming excessive, a part of the culture solution may be extracted together with the cells, and the same amount of a fresh culture medium may be added to reduce the viable cell density (cell bleeding).

The kind of the cell in the present invention is not particularly limited; however, the cell is preferably an animal cell and more preferably a mammalian cell. The cell may be a primary cell or a cell established as a cell line.

Examples of the cell include a Chinese hamster ovary (CHO) cell, a BHK cell, a 293 cell, a myeloma cell (such as an NS0 cell), PerC6 cell, a SP2/0 cell, a hybridoma cell, a COS cell (a cell derived from kidney of African green monkey), a 3T3 cell, a HeLa cell, a Vero cell (a renal epithelial cell of African green monkey), a MDCK cell (a cell derived from the canine kidney renal tubular epithelial cell), a PC12 cell, and a WI38 cell. Among these, particularly a CHO cell, a BHK cell, a 293 cell, a myeloma cell (such as an NS0 cell), a PerC6 cell, a SP2/0 cell, or a hybridoma cells is preferable, and a CHO cell is more preferable. The CHO cell is widely used for production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

These cells may be cells into which a foreign gene encoding a protein desired to be expressed has been introduced.

An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of cytomegalovirus (CMV), an early promoter of SV40, a retrovirus promoter, a metallothionein promoter, a heat shock promoter, an SRα promoter, and a promoter and enhancer of moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

In the present invention, the average cell size of cells is preferably 12 μm or more. In a case of cells having an average cell size of 12 μm or more, oxygen deficiency is likely to be remarkable around cells due to the influence of the overlap of layers (boundary layers) in which the culture solution stays, and thus the culture method for cells according to the embodiment of the present invention is useful particularly in the culture of cells, in which the average cell size of the cells is 12 μm or more.

In the present invention, during cell culture, the concentration of lactic acid in the culture solution is preferably less than 1.2 g/L, more preferably less than 1.0 g/L, still more preferably less than 0.8 g/L, and particularly preferably less than 0.6 g/L. The concentration of lactic acid in the culture solution can be measured using a commercially available product such as BioProfile 400 manufactured by Nova Biomedical Corporation.

In the present invention, during the cell culture, the concentration of the lactate dehydrogenase in the culture solution is preferably less than 2,000 U/L, more preferably less than 1,800 U/L, still more preferably less than 1,500 U/L, and particularly preferably 1,200 U/L or less. The concentration of the lactate dehydrogenase in the culture solution can be measured using a commercially available product such as Cedex Bio manufactured by Roche Diagnostics.

<Production method for useful substance>

The present invention relates to a production method for a useful substance, which includes culturing cells by the above-described culture method for cells according to the embodiment of the present invention to cause the cells to produce a useful substance.

In the present invention, the kind of useful substance is not particularly limited; however, the useful substance is preferably a recombinant protein. Examples of the useful substance include a recombinant polypeptide chain, a recombinant secreted polypeptide chain, an antigen-binding protein, a human antibody, a humanized antibody, a chimeric antibody, a mouse antibody, a bispecific antibody, an Fc fusion protein, a fragmented immunoglobulin, and a single-chain antibody (scFv).

The useful substance is preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culture by culturing a recombinant cell transformed with the above vector and expressing the incorporated DNA.

The bispecific antibody is an antibody which recognizes two kinds of antigenic specificity different from each other and which is prepared by a chemical method or cell fusion. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other, and the like.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

It is possible to purify a useful substance that is produced by the above-described culture. For the separation and purification of the useful substance, the separation and purification methods that are used for general proteins may be used. For example, it is possible to carry out separation and purification of a useful substance by appropriately selecting and combining means such as chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the useful substance obtained as above can be measured according to absorbance measurement, enzyme-linked immunosorbent assay (ELISA), or the like.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify a useful substance or partially remove a peptide by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The present invention will be further specifically described with reference to Examples; however, the present invention is not limited by Examples.

Examples

<Establishment of antibody-producing cell>

A vector containing a nucleic acid sequence encoding each of IgG1 and IgG4 was constructed, and the constructed vector was introduced into a CHO-DG44 cell to prepare a CHO-DG44 cell expressing IgG1 (an IgG1 cell) and a CHO-DG44 cell expressing IgG4 (an IgG4 cell). The construction of the vector and the introduction thereof into the cell were carried out according to Example 2 of JP2016-517691A. As described above, CHO cells producing monoclonal antibodies were prepared.

<Culture method for cells>

The above-described CHO cells were used to carry out cell culture according to a perfusion culture method. CD OptiCHO (manufactured by Thermo Fisher Scientific, Inc.) was used as a culture medium, and the seeding density of the cells was set to $5 \times 10^5$ cells/mL. The culture environment was maintained so that the pH was 6.7 or more and 7.4 or less, the culture temperature was 36°C or more and 38°C or less, and the $CO_2$ concentration was 25% or less. After the second day or later from the start of the culture, the filtered culture solution was extracted from the culture container, and a fresh culture medium was supplied.

In a case where the culture volume was 1 L or less, a glass container having a diameter of 114 mm was used as the culture container, a paddle-shaped stirring blade having a diameter of 85 mm was installed, and a predetermined amount of the culture solution was charged into the culture container to carry out culture. For filtration, an RF02PES membrane was used in an ATF2 system manufactured by Repligen Corporation.

In a case where the culture volume was more than 1 L and 3 L or less, a glass container having a diameter of 143 mm was used as the culture container, a propeller-shaped stirring blade having a diameter of 48 mm was installed, and for filtration, an RF02PES membrane was used in an ATF2 system manufactured by Repligen Corporation.

In a case where the culture volume was more than 3 L and 50 L or less, a plastic single-use bag having a diameter of 350 mm was used as the culture container, a propeller-shaped stirring blade having a diameter of 116 mm was installed, and a single-use system of ATF4 manufactured by Repligen Corporation was used for filtration.

In a case where the culture volume was more than 50 L and 500 L or less, a plastic single-use bag having a diameter of 756 mm was used as the culture container, a propeller-shaped stirring blade having a diameter of 251 mm was installed, and a single-use system of ATF10 manufactured by Repligen Corporation was used for filtration.

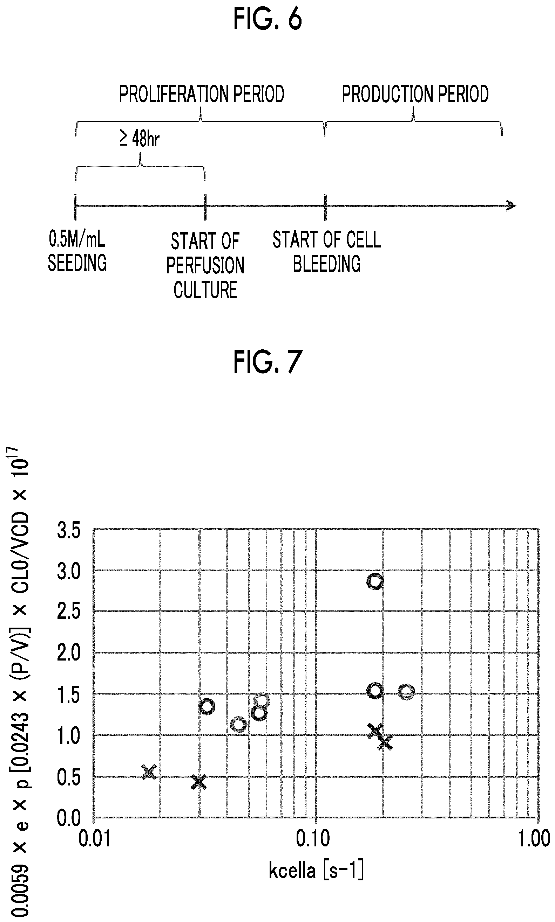

After reaching a targeted viable cell density, the cell suspension was extracted from the culture tank (cell bleeding), and the same amount of a fresh culture medium was supplemented at a frequency of at least once a day or continuously so that the targeted viable cell density was maintained, whereby the viable cell density was maintained while a predetermined culture solution amount being maintained. The period from the start of the culture to the first start of cell bleeding was defined as the cell proliferation period, and the period after the first start of cell bleeding was defined as the antibody production period (Fig. 6). The culture solution was sampled every day, and the viable cell density, the concentration of lactic acid in the culture solution, and the bleb formation rate were measured by the methods described below. In addition, a permeated culture solution after filtering was sampled, and the concentration of the antibody contained in the permeated culture solution and the quality of the antibody were measured and evaluated.

<Evaluation method and determination>

The viable cell density was measured using a Vi-CELL XR manufactured by Beckman Coulter Inc.

For the determination for the cell proliferation, in the proliferation period, A was assigned in a case where a ratio of the viable cell density to that of the previous day was 1.25 times or more, B was assigned in a case where the ratio thereof was 1.1 times or more and less than 1.25 times, and C was assigned in a case where the ratio thereof was less than 1.1 times, and in the antibody production period, A was assigned in a case where the ratio of the viable cell density to that of the previous day was 1.15 times or more, B was assigned in a case where the ratio thereof was 1.05 times or more and less than 1.15 times, and C was assigned in a case where the ratio thereof was less than 1.05 times.

In cell culture, the concentration of lactic acid is generally one of the representative indicators of oxygen deficiency. The concentration of lactic acid in the culture solution was measured using a BioProfile 400 manufactured by Nova Biomedical Corporation.

In the cell proliferation period, the concentration of lactic acid was not used to determine the presence or absence of oxygen deficiency.

In the antibody production period, A was assigned in a case where the concentration of lactic acid was less than 0.6 g/L, B was assigned in a case where the concentration thereof was 0.6 g/L or more and less than 1.2 g/L, and C was assigned in a case where the concentration thereof was 1.2 g/L or more.

Breb formation is known as one of the characteristics of cell apoptosis, and vesicle-shaped protrusions are formed on the surface of the cell membrane. In the studies so far, it has been found that the bleb formation rate increases in a case where cells being cultured are in a state of oxygen deficiency.

For the bleb formation rate, the total number of cells and the number of cells having vesicles on the cell surface were respectively counted in 10 or more microscopic images of a cell suspension which had been randomly captured in a visual field range of 0.3 mm or more and less than 3 mm to calculate the proportion of the number of cells having formed vesicles to the total number of cells.

A was assigned in a case where the bleb formation rate was less than 5%, B was assigned in a case where the bleb formation rate was 5% or more and less than 20%, and C was assigned in a case where the bleb formation rate was 20% or more.

Regarding the presence or absence of oxygen deficiency occurrence, the determinations for the viable cell density, the concentration of lactic acid, and the bleb formation rate were comprehensively evaluated, and it was determined that oxygen deficiency occurred in a case where B and C each were assigned one time or more or in a case where C was assigned two times or more.

<Measurement and evaluation of concentration of antibody contained in permeated culture solution and antibody quality>

The antibody concentration in the permeated culture solution was measured using Cedex Bio manufactured by Roche Diagnostics.

The quality of the antibody in the permeated culture solution was checked by measuring and evaluating the antibody purity, the sugar chain, and the charge.

Purity was evaluated by two methods.

The first method was carried out using a column of TSKgel G3000WXL (7.8 mm × 300 mm) manufactured by Tosoh Corporation. The evaluation was carried out assuming that the proportion of the maximum peak area to the total peak area at 4 to 11 minutes of the chromatogram corresponded to a monomer.

The second method was carried out using CE PA800 plus manufactured by SCIEX. The proportion of the heavy chain portion area to the total area was calculated in the result of the non-reducing electropherogram.

The sugar chain was evaluated with each of G0F, G1F, and Man5, which are indicators of antibody maturity. The measurement was carried out using a column of ODS HYPERSIL 3 $\mu$m (2.1 mm × 150 mm) manufactured by Thermo Fisher Scientific, Inc. The proportion of each of the peak areas of G0F, G1F, and Man5 to the total peak area of the chromatogram at 22 to 46 minutes was calculated.

The charge measurement was carried out using a ProPac WCX 5 $\mu$m (4 mm × 250 mm) column manufactured by Thermo Fisher Scientific, Inc. The maximum peak in the chromatogram at 10 to 60 minutes was denoted as Neutral, a peak that appeared before Neutral was denoted as Acidic, and a peak that appeared after Neutral was denoted as Basic, and the proportion of each peak area to the total peak area was calculated.

<Measuring method for kcella>

In a steady culture state (a steady state) in which a culture solution is continuously subjected to stirring and oxygen supply to maintain a dissolved oxygen concentration of a predetermined set value CLO in the culture solution for 24 hours or more, the oxygen supply was stopped, and this moment was defined as a temporal starting point (t = 0).

A change between the dissolved oxygen concentration CL0 at the starting point and the dissolved oxygen concentration CL in the solution were measured every 3 seconds for 60 seconds from the starting point, and Ln(CL/CL0) was calculated. Ln(CL/CL0) was plotted with respect to the elapsed time t, from which a slope was obtained as -kcella by linear approximation.

<Calculation method for turbulence energy dissipation rate $\varepsilon$>

The turbulence energy dissipation rate $\varepsilon$ can be calculated by using the commercially available software in computational fluid dynamics (CFD). In the present Example, the calculation was carried out using Fluent manufactured by ANSYS Inc. The mass conservation equation, the momentum conservation equation, the transport equation of the k-$\varepsilon$ model, and the improved wall processing $\varepsilon$ equation were combined, where these were in terms of the software standard. Meshes that mimicked the shape of the culture tank, the shape of the stirring blade, and the predetermined amount of liquid were created, the fluid viscosity, the fluid density, the stirring rotation speed, the rotation angle, the direction of gravitational force, and the liquid-surface boundary condition were set as parameters, and a convergence calculation was carried out to obtain a calculation result of the turbulence energy dissipation rate. It is noted that "creating meshes" means, generally in a case of carrying out numerical analysis, to divide an analysis target into elements in a case of dividing an analysis target on software into elements having a simple shape, applying a model or expression specified for each element to solve a calculation, and integrating the calculation result of each of the elements to acquire the analysis result of the entire analysis target.

<Evaluation method for cell damage>

Cell damage was evaluated by measuring lactate dehydrogenase (LDH) in the culture solution. For the measurement, a sampled culture solution was centrifuged at 300 G × 5 minutes, and the supernatant was subjected to measurement using Cedex Bio manufactured by Roche Diagnostics.

A was assigned for a value of LDH less than 2,000 U/L (a state without no problem), B was assigned for a value of 2,000 U/L or more and less than 3000 U/L (a state unfavorable for practical use), and C was assigned for a value of 3,000 U/L or more (a state in which it is difficult to continue culture).

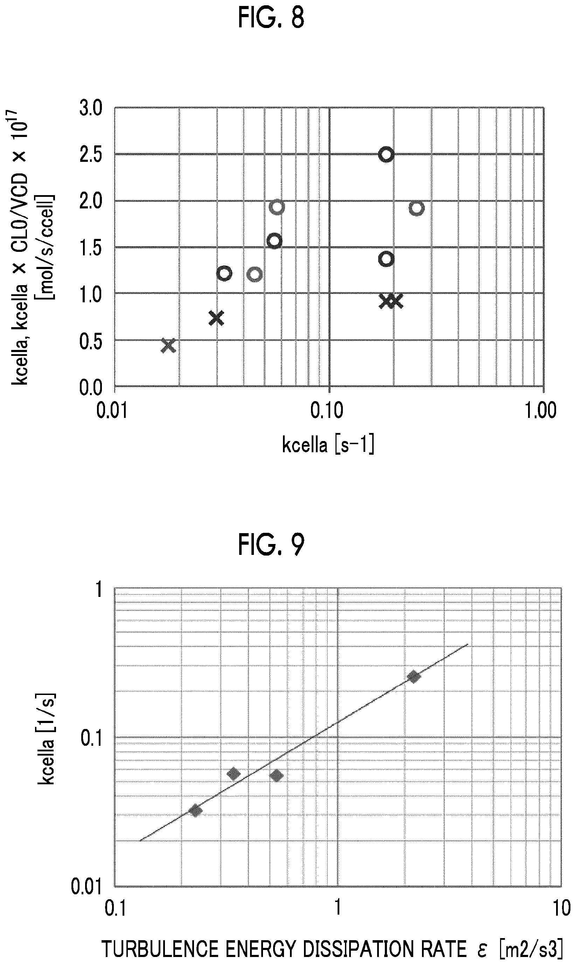

The results of the above measurements and evaluations are shown in Tables 1 to 4 and Figures 7 to 9.

As shown in Table 2, in a case where the condition of Expression 1 or the condition of Expression 2 according to the present invention was satisfied (Examples 1 to 7), oxygen deficiency did not occur. However, in a case where the condition of Expression 1 and the condition of Expression 2 were not satisfied (Comparative Examples 1 to 4), oxygen deficiency occurred.

[Table 1]

| | Viable cell density VCD [cells/mL] | Dissolved oxygen concentration CL0 $\times 10^8$ [mol/mL] | Culture volume [L] | Stirring power P/V [W/m$^3$] | Turbulence energy dissipation rate $\varepsilon$ [m$^2$/s$^3$] | Kcella [1/s] | Oxygen deficiency occurrence |
|---|---|---|---|---|---|---|---|
| Example 1 | $120 \times 10^6$ | 0.90 | 0.83 | 145.5 | 2.186 | 0.256 | Absent |
| Example 2 | $120 \times 10^6$ | 3.38 | 1.6 | 83.7 | 0.531 | 0.056 | Absent |
| Example 3 | $120 \times 10^6$ | 4.05 | 50 | 80.6 | 0.340 | 0.057 | Absent |
| Example 4 | $120 \times 10^6$ | 4.50 | 500 | 74.2 | 0.230 | 0.032 | Absent |
| Example 5 | $160 \times 10^6$ | 4.27 | 50 | 80.8 | - | 0.045 | Absent |
| Example 6 | $200 \times 10^6$ | 1.49 | 0.83 | 146.4 | - | 0.185 | Absent |
| Example 7 | $300 \times 10^6$ | 4.05 | 0.83 | 147.4 | - | 0.185 | Absent |
| Comparative Example 1 | $60 \times 10^6$ | 1.49 | 2.0 | 44.7 | - | 0.030 | Present |
| Comparative Example 2 | $60 \times 10^6$ | 1.49 | 50 | 58.5 | - | 0.018 | Present |
| Comparative Example 3 | $100 \times 10^6$ | 0.45 | 0.83 | 145.3 | - | 0.204 | Present |
| Comparative Example 4 | $300 \times 10^6$ | 1.49 | 0.83 | 147.4 | - | 0.185 | Present |

[Table 2]

| | Expression $2 \times 10^{17}$ (kcella) | Expression $1 \times 10^{17}$ (P/V) | Determination for cell proliferation | Determination for concentration of lactic acid | Determination for bleb formation rate | Oxygen deficiency occurrence |
|---|---|---|---|---|---|---|
| Example 1 | 1.92 | 1.52 | A | B | B | Absent |
| Example 2 | 1.57 | 1.27 | A | A | A | Absent |
| Example 3 | 1.93 | 1.41 | A | A | A | Absent |
| Example 4 | 1.22 | 1.34 | A | A | B | Absent |
| Example 5 | 1.2 | 1.12 | A | B | A | Absent |
| Example 6 | 1.37 | 1.54 | A | A | A | Absent |
| Example 7 | 2.49 | 2.86 | A | B | A | Absent |
| Comparative Example 1 | 0.74 | 0.74 | C | - | C | Present |
| Comparative Example 2 | 0.44 | 0.61 | B | - | C | Present |
| Comparative Example 3 | 0.92 | 0.91 | C | C | C | Present |

(continued)

| | Expression $2 \times 10^{17}$ (kcella) | Expression $1 \times 10^{17}$ (P/V) | Determination for cell proliferation | Determination for concentration of lactic acid | Determination for bleb formation rate | Oxygen deficiency occurrence |
|---|---|---|---|---|---|---|
| Comparative Example 4 | 0.91 | 1.05 | C | C | A | Present |

[Table 3]

| Evaluation results of productivity and quality | | | | | | | | |
|---|---|---|---|---|---|---|---|---|
| | Antibody concentration | Purity | | Sugar chain | | | Charge | | |
| | | Monomer | Heavy chain | GOF | G1F | Man5 | Neutral | Acidic | Basic |
| Example 4 | 1.6 g/L | 98% | 95% | 70% | 14% | 3% | 58% | 46% | 8% |

[Table 4]

| kcella and cell damage | | | | |
|---|---|---|---|---|
| | Culture volume [L] | Stirring power P/V [W/m$^3$] | LDH [U/L] | Cell damage |
| Example 4 | 500 | 74.2 | 1,200 | A |
| Example 1 | 0.83 | 145.5 | 1,750 | A |
| Comparative Example 5 | 0.83 | 2026 | 2,100 | B |

**Claims**

1. A culture method for cells, in which a relationship between a stirring power P/V per unit volume, a dissolved oxygen concentration CL0, and a viable cell density VCD satisfies a condition of Expression 1, in cell culture in which the viable cell density is $60 \times 10^6$ cells/mL or more,

$$\text{Expression 1: } 0.0059 \times \exp[0.0243 \times (P/V)] \times CL0/VCD \geq 1.10 \times 10^{-17}$$

in the expression,

CLO indicates a dissolved oxygen concentration [mol/mL];
VCD indicates a viable cell density [cells/mL]; and
P/V indicates a stirring power [W/m$^3$] per unit volume, which is defined by the following expression;

$$P/V = \rho \times (n/60)^3 \times Di^5/V \times Np$$

in the expression,
$\rho$ indicates a density of a culture solution [kg/m$^3$];
n indicates a stirring rotation speed [rpm];
Di indicates a stirring blade diameter [m];
V indicates a culture solution amount [m$^3$]; and
Np indicates a stirring power coefficient.

2. The culture method for cells according to claim 1,
wherein the stirring power P/V per unit volume satisfies, $60 \leq P/V \leq 2,000$.

3. The culture method for cells according to claim 1 or 2,
   wherein the stirring rotation speed n satisfies, $120 \leq n \leq 600$.

4. The culture method for cells according to any one of claims 1 to 3,
   wherein the relationship between the stirring power P/V per unit volume, the dissolved oxygen concentration CL0, and the viable cell density VCD satisfies a condition of $0.0059 \times \exp[0.0243 \times (P/V)] \times CLO/VCD \leq 4.00 \times 10^{17}$.

5. The culture method for cells according to any one of claims 1 to 4,
   wherein a cell culture period satisfying the condition of Expression 1 is 20 days or more.

6. The culture method for cells according to any one of claims 1 to 5,
   wherein the condition of Expression 1 is satisfied over an entire culture period of cells.

7. A culture method for cells, in which a relationship between kcella defined as below, a dissolved oxygen concentration CLO in a steady state, and a viable cell density VCD satisfies a condition of Expression 2, in cell culture in which the viable cell density is $60 \times 10^6$ cells/mL or more,

$$\text{Expression 2: } kcella \times CL0/VCD \geq 1.00 \times 10^{-17} \text{ [mol/s/cell]}$$

in the expression,

    CLO indicates a dissolved oxygen concentration [mol/mL];
    VCD indicates a viable cell density [cells/mL]; and
    the kcella indicates an overall oxygen transfer capacity coefficient [/s] from a culture medium to cells, which is defined according to $\text{Ln}(CL/CLO) = -kcella \times t$,
    in the expression,
    -kcella is defined as a slope which is obtained by defining a steady state as a state in which a culture solution is continuously subjected to stirring and oxygen supply to maintain a dissolved oxygen concentration of a predetermined set value CL0 in the culture solution for 24 hours or more, defining a temporal starting point as a moment at which the oxygen supply is stopped while the stirring is continued from the steady state, measuring a change between a dissolved oxygen concentration CLO at the starting point and a dissolved oxygen concentration CL in the solution for 60 seconds from the starting point, and subjecting a change in $\text{Ln}(CL/CLO)$ with respect to an elapsed time t to linear approximation according to a least square method.

8. The culture method for cells according to claim 7,
   wherein the kcella satisfies, $0.03 \leq kcella \leq 0.39$.

9. The culture method for cells according to claim 7 or 8,
   wherein the relationship between the kcella, the dissolved oxygen concentration CLO in the steady state, and the viable cell density VCD satisfies a condition of $kcella \times CLO/VCD \leq 4.00 \times 10^{-17}$ [mol/s/cell].

10. The culture method for cells according to any one of claims 7 to 9,
    wherein culture is controlled such that the condition of Expression 2 is satisfied by grasping, in advance in a target culture tank and at a target liquid amount, a correlation between a calculated value of a turbulence energy dissipation rate $\varepsilon$ of the culture solution and an actually measured value of the kcella in a case where a stirring rotation speed is changed and increasing a stirring power in association with cell proliferation to increase kcella obtained from the correlation.

11. The culture method for cells according to any one of claims 7 to 9,
    wherein culture is controlled such that the condition of Expression 2 is satisfied by increasing at least one of the kcella or the CLO in association with an increase of the VCD.

12. The culture method for cells according to any one of claims 7 to 11,
    wherein a cell culture period satisfying the condition of Expression 2 is 20 days or more.

13. The culture method for cells according to any one of claims 7 to 12,
    wherein the condition of Expression 2 is satisfied over an entire culture period of cells.

14. The culture method for cells according to any one of claims 1 to 13,
wherein the CLO satisfies, $0.45 \times 10^{-8} \leq CLO \leq 6.75 \times 10^{-8}$.

15. The culture method for cells according to any one of claims 1 to 14,
wherein the viable cell density is $80 \times 10^6$ cells/mL or more.

16. The culture method for cells according to any one of claims 1 to 15,
wherein the culture solution amount is 50 L or more.

17. The culture method for cells according to any one of claims 1 to 16,
wherein the culture method for cells is perfusion culture.

18. The culture method for cells according to any one of claims 1 to 17,
wherein the cells are mammalian cells.

19. The culture method for cells according to claim 18,
wherein the mammalian cell is a Chinese hamster ovary cell.

20. The culture method for cells according to any one of claims 1 to 19,
wherein an average cell size of the cells is 12 $\mu$m or more.

21. The culture method for cells according to any one of claims 1 to 20,
wherein during the cell culture, a concentration of lactic acid in a culture solution is less than 1.2 g/L.

22. The culture method for cells according to any one of claims 1 to 21,
wherein during the cell culture, a concentration of lactate dehydrogenase in a culture solution is less than 2,000 U/L.

23. A production method for a useful substance, comprising:
culturing cells by the culture method for cells according to any one of claims 1 to 22 to cause the cells to produce a useful substance.

24. The production method for a useful substance according to claim 23,
wherein the useful substance is a recombinant protein.

FIG. 1

INDICATOR IN RELATED ART:
OXYGEN TRANSFER CAPACITY COEFFICIENT kLa IN TERMS OF TRANSFER FROM GAS TO CULTURE MEDIUM (SOLUTION)

INDICATOR ACCORDING TO PRESENT INVENTION:
OXYGEN TRANSFER CAPACITY COEFFICIENT kcella IN TERMS OF TRANSFER FROM CULTURE MEDIUM TO CELL

OXYGEN

DISSOLUTED INTO CULTURE MEDIUM

INCORPORATED INTO CELL

AIR BUBBLE          IN CULTURE MEDIUM          CELL

OXYGEN CONCENTRATION CL

kcella LARGE

SLOPE ≒ OXYGEN TRANSFER AMOUNT

CL0

kcella SMALL

Ccell

BOUNDARY LAYER (THICKNESS $\delta$)

LIQUID FLOW (TURBULENT)

TRANSFER COEFFICIENT: kcella $\propto 1/\delta$

EP 4 310 175 A1

# FIG. 2

# FIG. 3

FIG. 4

# FIG. 5

STEADY CULTURE STATE

MEASUREMENT OF
DISSOLVED OXYGEN
CONCENTRATION

OXYGEN
SUPPLY

STOPPING OF OXYGEN SUPPLY
→ MEASUREMENT OF OXYGEN
CONCENTRATION

MEASUREMENT OF
DISSOLVED OXYGEN
CONCENTRATION

60s          t

$\ln (CL/C0)$

SLOPE FROM
EXPRESSION
SUBJECTED
TO LINEAR
APPROXIMATION:
−kcella

# FIG. 6

PROLIFERATION PERIOD    PRODUCTION PERIOD

$\geq$ 48hr

0.5M/mL
SEEDING

START OF
PERFUSION
CULTURE

START OF CELL
BLEEDING

# FIG. 7

$0.0059 \times e \times p\,[0.0243 \times (P/V)] \times CL0/VCD \times 10^{17}$

kcella [s-1]

## FIG. 8

## FIG. 9

**EP 4 310 175 A1**

## INTERNATIONAL SEARCH REPORT

| International application No. |
|---|
| **PCT/JP2022/011766** |

**A.    CLASSIFICATION OF SUBJECT MATTER**

*C12N 5/071*(2010.01)i; *C12N 5/10*(2006.01)i; *C12N 1/00*(2006.01)i; *C12P 21/00*(2006.01)i
FI:    C12N5/071; C12N5/10; C12N1/00 B; C12N1/00 D; C12P21/00 C

According to International Patent Classification (IPC) or to both national classification and IPC

**B.    FIELDS SEARCHED**

Minimum documentation searched (classification system followed by classification symbols)

C12N5/071; C12N5/10; C12N1/00; C12P21/00; C12M1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| A | JP 8-38166 A (TABAI ESPEC CORPORATION) 13 February 1996 (1996-02-13)<br>    claim 1, paragraph [0033], fig. 1, 3 | 1-24 |
| A | CN 201678670 U (NANJING UNIVERSITY OF TECHNOLOGY) 22 December 2010 (2010-12-22)<br>    claim 1, paragraphs [0007], [0010], [0035] | 1-24 |
| A | JP 2015-216886 A (YOKOGAWA ELECTRIC CORPORATION) 07 December 2015 (2015-12-07)<br>    claim 1, paragraphs [0014], [0021], fig. 1 | 1-24 |

☐ Further documents are listed in the continuation of Box C.    ☑ See patent family annex.

| *    Special categories of cited documents: | "T"    later document published after the international filing date or priority date and not in conflict with the application but cited to understand the principle or theory underlying the invention |
|---|---|
| "A"    document defining the general state of the art which is not considered to be of particular relevance | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **19 May 2022** | **31 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

23

**INTERNATIONAL SEARCH REPORT**

| International application No. |
|---|
| **PCT/JP2022/011766** |

**Box No. III    Observations where unity of invention is lacking (Continuation of item 3 of first sheet)**

This International Searching Authority found multiple inventions in this international application, as follows:

Document 1: JP 8-38166 A (TABAI ESPEC CORPORATION) 13 February 1996 (1996-02-13), claim 1, paragraph [0033], fig. 1, 3 (Family: none)

The claims are classified into the following two inventions.

Invention 1: Claims 1-6 and parts of claims 14-24 wherein "an agitation power P/V per unit volume, a dissolved oxygen level CL0 and a viable cell density VCD fulfill the conditions in formula 1"

Said claims have the special technical feature of a "cell culture having a viable cell density of $60 \times 10^6$ cells/mL or more, wherein an agitation power P/V per unit volume, a dissolved oxygen level CL0 and a viable cell density VCD fulfill the conditions in formula 1", and thus are classified as invention 1.

Invention 2: Claims 7-13 and parts of claims 14-24 wherein "kcella, a dissolved oxygen level CL0 at steady state and a viable cell density VCD fulfill the conditions in formula 2"

Said claims and claim 1 classified as invention 1 share the technical feature of a "cell culture having a viable cell density of $60 \times 10^6$ cells/mL or more". However, said technical feature does not make a contribution over the prior art in light of the disclosures in document 1 (particularly, see paragraph [0033]), and therefore cannot be said to be a special technical feature. Moreover, there are no other same or corresponding special technical features among said claims and claim 1.

Furthermore, said claims are not dependent on claim 1. In addition, said claims are not substantially identical to or similarly closely related to any of the claims classified as invention 1.

Said claims therefore cannot be classified as invention 1.

Said claims have the special technical feature of a cell culture having a viable cell density of $60 \times 10^6$ cells/mL or more, wherein kcella, a dissolved oxygen level CL0 at steady state and a viable cell density VCD fulfill the conditions in formula 2", and thus are classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

INTERNATIONAL SEARCH REPORT
Information on patent family members

International application No.

**PCT/JP2022/011766**

| Patent document cited in search report | | | Publication date (day/month/year) | Patent family member(s) | Publication date (day/month/year) |
|---|---|---|---|---|---|
| JP | 8-38166 | A | 13 February 1996 | (Family: none) | |
| CN | 201678670 | U | 22 December 2010 | (Family: none) | |
| JP | 2015-216886 | A | 07 December 2015 | US 2015/0329817 A1 claim 1, paragraphs [0016], [0027], [0028], fig. 1 EP 2947140 A1 | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011092117 A
- JP 2016517691 A